# EUROPÄISCHE PATENTSCHRIFT

(11) **EP 0 770 366 B1**
(45) Veröffentlichungstag und Bekanntmachung des Hinweises auf die Patenterteilung: **27.08.1997**
(21) Anmeldenummer: 96111896.5
(22) Anmeldetag: 24.07.1996
(51) Int. Cl.: A61F 2/06, A61M 29/00

(54) **Vorrichtung zur Applikation einer Gefässstütze**
Device for the application of a vessel support
Dispositif pour l'application d'un support vasculaire

(30) Priorität: 27.10.1995 DE 19540084
(43) Veröffentlichungstag der Anmeldung: 02.05.1997
(73) Patentinhaber: Figulla, Hans-Reiner, Prof. Dr., 37085 Göttingen (DE)
(72) Erfinder: Figulla, Hans-Reiner, Prof. Dr., 37085 Göttingen (DE)
(74) Vertreter: Selting, Günther, Dipl.-Ing.

(56) Entgegenhaltungen:
- EP-A- 0 364 787
- EP-A- 0 592 726
- EP-A- 0 611 556
- US-A- 5 258 020

## Beschreibung

Die Erfindung betrifft eine Vorrichtung zur Applikation einer bleibend aufweitbaren Gefäßstütze, mit einem Ballonkatheter mit einem Ballonabschnitt, auf dem die Gefäßstütze im komprimierten Zustand sitzt wie es im Oberbegriff des Anspruchs 1 definiert ist. Eine solche Vorrichtung ist z.B. aus der EP-A-0 364 787 bekannt.

Zur Rekanalisation von verengten oder verschlossenen Koronararterien wird als Standardeingriff die perkutane Ballondilatation eingesetzt. Bekannt sind Gefäßstützen aus Drahtgeflecht, die in das Blutgefäß eingebracht werden, um dieses endoluminal zu schienen. Eine solche Gefäßstütze ist beschrieben in EP 0 282 175 B1. Die Gefäßstütze sitzt in komprimiertem Zustand auf dem Ballonabschnitt eines Ballonkatheters. Der Ballonkatheter wird in das Gefäß eingebracht und der Ballonabschnitt durch Inflation aufgeweitet. Dabei wird die Gefäßstütze bleibend expandiert, um die Gefäßwand abzustützen. Nach Deflation wird der Ballonkatheter entfernt, während die Gefäßstütze im Blutgefäß verbleibt.

Um Beschädigungen des Gefäßes durch die Gefäßstütze beim Einführen des Ballonkatheters zu vermeiden, ist es ferner bekannt, einen Schutzkatheter über die auf dem Ballonkatheter sitzende Gefäßstütze zu schieben (US-A-5 195 984 oder EP-A-0 364 787). Die Gefäßstütze wird mit dem umgebenden Schutzkatheter in dem betreffenden Gefäßsegment positioniert. Anschließend wird der Schutzkatheter so weit hinter die Gefäßstütze zurückgezogen, daß die Gefäßstütze frei an der Implantationsstelle liegt. Dann erfolgt die Implantation der Gefäßstütze durch Expansion des Ballonabschnitts. Durch den Schutzkatheter wird der Durchmesser der gesamten Vorrichtung vergrößert und die Flexibilität eingeschränkt. Insbesondere in engen und gekrümmten Gefäßverläufen kann eine solche Vorrichtung aus Ballonkatheter und Schutzkatheter nicht eingesetzt werden. Ferner ist vor der Implantation der Gefäßstütze eine Vordilatation des Gefäßabschnittes erforderlich. Diese erfolgt mit einem zusätzlichen Ballonkatheter. Daher bevorzugen viele Anwender eine andere Vorgehensweise: Zunächst wird mit einem Ballonkatheter vordilatiert, anschließend die Gefäßstütze auf den zuvor benutzten Ballon aufgezogen und komprimiert. Danach erfolgt die Implantation der Gefäßstütze durch Aufweiten des Ballonabschnitts, ohne daß ein zusätzlicher Schutzkatheter verwendet würde. Bei dieser Vorgehensweise besteht jedoch die Gefahr, daß die Gefäßstütze auf dem Ballonabschnitt verrutschen kann, oder z.B. an einer Verkalkung im Gefäß vom Ballonkatheter gestreift wird.

In EP 0 707 837 A1, die gemäß Art. 54 (3) EPÜ als Stand der Technik gilt, ist ein Ballonkatheter beschrieben, der zwei hintereinander angeordnete Ballonabschnitte aufweist, wobei der proximale Ballonabschnitt durch zwei Stützteile begrenzt ist. Dieser Ballonabschnitt wird von einer Ballonhaut gebildet, die auch die Stützteile umschließt. Die Stützteile sind hierbei Ringe von rechteckigem Querschnitt, also ohne Kegelstumpfform.

Eine weitere Vorrichtung ist aus WO 93/15787 bekannt. Diese Vorrichtung weist einen Ballonkatheter auf, der an beiden Enden des Ballonabschnitts mit ringförmigen Stützteilen versehen ist, welche die komprimierte Gefäßstütze daran hindern, sich von dem Ballonabschnitt zu entfernen. Der Katheterschlauch ist an seinem distalen Ende, welches das distale Stützteil überragt, kegelstumpfförmig verjüngt, um einen glatten Übergang zu einem austretenden Führungsdraht zu schaffen. Der kegelstumpfförmige Bereich erfaßt jedoch nicht das distale Stützteil.

Der Erfindung liegt die Aufgabe zugrunde, eine Vorrichtung zur Applikation einer Gefäßstütze zu schaffen, die eine sichere Implantation der Gefäßstütze ohne zusätzlichen Schutzkatheter ermöglicht.

Die Lösung dieser Aufgabe erfolgt erfindungsgemäß mit den im Patentanspruch 1 angegebenen Merkmalen.

Bei der erfindungsgemäßen Vorrichtung ist an beiden Enden des Ballonabschnitts jeweils ein die komprimierte Gefäßstütze abschirmendes Stützteil vorgesehen, das derart bemessen ist, daß es durch die aufgeweitete Gefäßstütze hindurchpaßt. Dieses Stützteil bildet beim Vorschieben der Gefäßstütze in dem Blutgefäß gewissermaßen einen Schild, der das in Schieberichtung vorn liegende Ende der Gefäßstütze radial überragt, so daß die Gefäßstütze nicht gegen Hindernisse stoßen kann. Die Gefäßstütze ist somit an ihrem vorderen Ende eingebettet. Von dem rückwärtigen (proximalen) Ende her wird die Gefäßstütze von dem weiteren Stützteil abgeschirmt und gegen Zurückgleiten auf dem Ballonkatheter gesichert.

Die Stützteile bestehen aus jeweils einem sich stetig erweiternden Ring, dessen dünneres Ende von dem Ballonabschnitt abgewandt ist. Auf diese Weise bilden die Stützteile Rampen, die ein hindernisfreies Durchschieben des Ballonkatheters durch das Blutgefäß ermöglichen. Die Stützteile sind so dimensioniert, daß sie die komprimierte Gefäßstütze - in axialer Richtung des Ballonkatheters gesehen - soeben abschirmen, so daß die Gefäßstütze die Stützteile nicht überragt. Die kegelstumpfförmige Ausbildung der Stützteile dient dazu, eine gute Passierbarkeit durch eine Stenose zu ermöglichen.

Dadurch, daß die Ballonhaut die Stützteile umschließt, wird das Aufblasen des Ballons vereinfacht, da die Ballonhaut von den kegelstumpfförmigen Stützteilen bereits angrenzend an den Ballonabschnitt aufgeweitet ist. Die Stützteile bilden somit auch Rampen für die Erleichterung des Aufblasens des Ballons. Ferner kann der Katheterschlauch auf einfache Weise als durchgehender Schlauch hergestellt werden, ohne daß es einer speziellen Formgebung im Bereich des Ballonabschnitts bedürfte. Der Ballonabschnitt wird dann durch nachträgliche Montage der Stützteile und der Ballonhaut an dem Katheterschlauch gebildet werden.

Der Ballonkatheter kann auch verwendet werden, um eine Vordilatation (ohne Gefäßstütze) vorzunehmen. Anschließend wird der Ballonkatheter aus dem Gefäß herausgezogen und die Gefäßstütze auf ihm befestigt. Dann wird der Ballonkatheter mit der Gefäßstütze in das Blutgefäß eingeführt und an der Implantationsstelle erfolgt das Aufblasen des Ballonabschnitts und die Aufweitung der Gefäßstütze.

Die Stützteile können aus röntgenkontrastgebendem Material bestehen und somit eine genaue Positionierung der Gefäßstütze unter Röntgenkontrolle ermöglichen.

Im folgenden wird unter Bezugnahme auf die Zeichnungen ein Ausführungsbeispiel der Erfindung näher erläutert.

Es zeigen:
- Fig. 1: einen Längsschnitt durch den Ballonkatheter,
- Fig. 2: die gleiche Darstellung wie Fig. 1, jedoch mit einer auf dem Ballonabschnitt sitzenden komprimierten Gefäßstütze und
- Fig. 3: eine Seitenansicht des Ballonkatheters mit einem Längsschnitt durch die aufgeweitete Gefäßstütze.

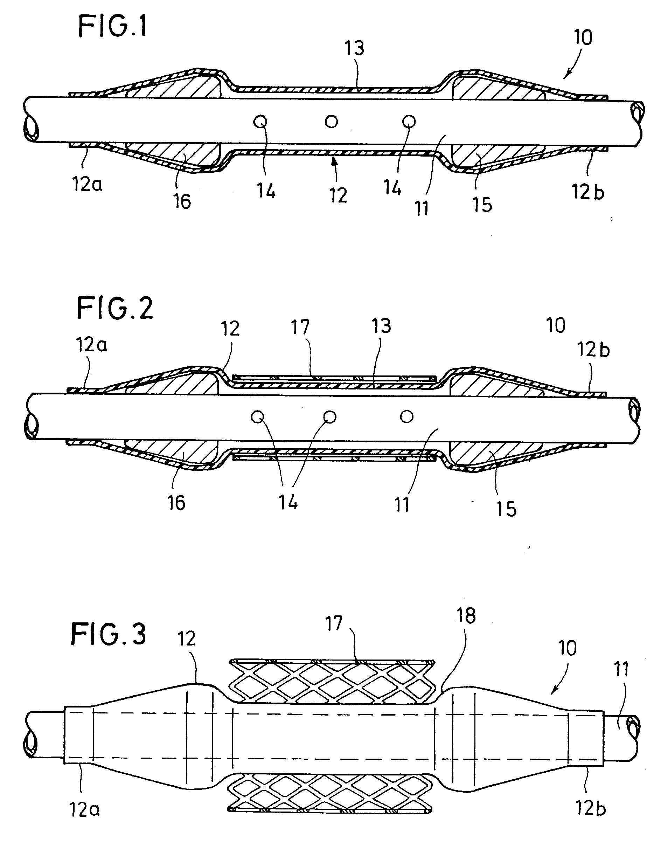

Gemäß Fig. 1 ist ein Ballonkatheter 10 vorgesehen, der einen Katheterschlauch 11 in Form eines langgestreckten Schlauches aufweist. Der Katheterschlauch 11 ist auf einem Längenabschnitt mit einer schlauchförmigen Ballonhaut 12 umgeben, die an ihren Enden 12a,12b fest und dicht mit dem Katheterschlauch 11 verbunden ist, während der zwischen den Enden 12a,12b liegende Bereich den Ballonabschnitt 13 bildet. Im Bereich des Ballonabschnitts 13 sind an dem Katheterschlauch 11 Löcher 14 vorgesehen, die mit dem Lumen des Katheterschlauchs in Verbindung stehen. Durch Anlegen von Druck an das Innere des Katheterschlauchs 11 wird der Ballonabschnitt 13 aufgeblasen.

An dem distalen (patientenseitigen) Ende des Ballonabschnitts 13 ist ein ringförmiges Stützteil 15 an dem Katheterschlauch 11 befestigt, z.B. durch Klebung, welches den Katheterschlauch umgibt. Das Stützteil 15 besteht aus einem generell kegelstumpfförmigen Ring, dessen dünneres Ende dem Ballonabschnitt 13 abgewandt ist.

In gleicher Weise ist ein Stützteil 16 am proximalen (patientenfernen) Ende des Ballonabschnitts 13 vorgesehen. Auch dieses Stützteil 16 ist ein kegelstumpfförmiger Ring, dessen dünneres Ende von dem Ballonabschnitt 13 abgewandt ist.

Die Ballonhaut 12 bedeckt die beiden Stützteile 15,16. Der gegenseitige Abstand der Stützteile 15,16 beträgt zwischen 10 und 30 mm. Der maximale Außendurchmesser eines Stützteils ist um 0,12 bis 0,6 mm größer als der Außendurchmesser des Katheterschlauchs 11.

In Fig. 2 ist die auf dem Ballonabschnitt 13 sitzende Gefäßstütze 17 zusätzlich dargestellt. Die Gefäßstütze 17 besteht aus einem rohrförmigen Drahtgeflecht oder einer anderen Drahtstruktur, die plastisch verformbar ist und denjenigen Zustand, in den sie gezwungen wurde, beibehält. Die Gefäßstütze 17 kann rohrförmig ausgebildet und dann in Längsrichtung auf den Ballonabschnitt 13 aufgeschoben sein. Es besteht aber auch die Möglichkeit, daß die Gefäßstütze 17 nach Art eines geschlitzten Rohres ausgebildet ist und dann seitlich über den Ballonabschnitt 13 geschoben wird. In jedem Fall wird die Gefäßstütze nach dem Aufbringen auf den Ballonabschnitt zusammengedrückt, z.B. durch Krimpen mit einem geeigneten Werkzeug. Die Gefäßstütze 18 im komprimierten Zustand füllt den Zwischenraum zwischen den beiden Stützteilen 15,16 in Längsrichtung im wesentlichen aus.

In dem in Fig. 2 dargestellten Zustand wird der Ballonkatheter 10 in das Blutgefäß eingeführt. Mindestens eines der Stützteile 15,16 enthält ein Röntgenkontrastmittel, z.B. Gold, Platin, Iridium oder Legierungen hieraus, so daß die Positionierung der Gefäßstütze 17 am Implantationsort unter Röntgenkontrolle möglich ist. Wenn die Gefäßstütze 17 am Implantationsort angelangt ist, wird der Ballonabschnitt 13 aufgeblasen, wobei er die Gefäßstütze 17 expandiert. Dies ist in Fig. 3 dargestellt. Wird anschließend der Druck aus dem Ballonabschnitt 13 abgelassen, zieht der Ballonabschnitt 13 sich wieder auf den ursprünglichen Zustand zusammen. Der Innendurchmesser der Gefäßstütze 17 ist nunmehr größer als der maximale Außendurchmesser der Stützteile 15,16, so daß der Ballonkatheter 10 aus der Gefäßstütze herausgezogen werden kann. Zweckmäßigerweise ist die Ringschulter 18 am rückwärtigen Ende des vorderen Stützteils 15 so abgerundet oder abgeschrägt, daß sie beim Zurückziehen des Ballonkatheters nicht an der Gefäßstütze festhaken kann.

Aus Fig. 3 ist auch erkennbar, daß die Gefäßstütze 17 aus einer Art Maschendraht oder Streckmetall besteht, das radial deformierbar ist. Selbstverständlich können auch Gefäßstützen mit anderen Drahtkonfigurationen benutzt werden, z.B. solche, die seitlich über dem Ballonabschnitt des Katheters 10 geschoben werden können.

## Patentansprüche

1. Vorrichtung zur Applikation einer bleibend aufweitbaren Gefäßstütze (17), mit einem Ballonkatheter (10), der an einem Katheterschlauch (11) einen Ballonabschnitt (13) zur Aufnahme der Gefäßstütze (17) im komprimierten Zustand und an beiden Enden des Ballonabschnitts (13) jeweils ein die komprimierte Gefäßstütze (17) abschirmendes Stützteil (15,16) aufweist,
wobei jedes Stützteil (15,16) aus einem kegelstumpfförmigen Ring besteht, dessen dünneres Ende von dem Ballonabschnitt (13) abgewandt ist, **dadurch gekennzeichnet,** daß die den Katheterschlauch (11) im Bereich des Ballonabschnitts (13) umgebende Ballonhaut (12) die Stützteile (15,16) umschließt.

2. Vorrichtung nach Anspruch 1, dadurch gekennzeichnet, daß mindestens eines der Stützteile (15,16) eine Röntgenkontrastierung aufweist.

3. Vorrichtung nach Anspruch 1 oder 2, dadurch gekennzeichnet, daß der Abstand zwischen den Stützteilen (15,16) 20 bis 30 mm beträgt.

4. Vorrichtung nach einem der Ansprüche 1-3, dadurch gekennzeichnet, daß der Durchmesser der Stützteile (15,16) um 0,12 bis 0,6 mm größer ist als derjenige des Ballonkatheters (10) außerhalb des Ballonabschnitts (13).

## Claims

1. Device for the application of a permanently expandable vessel support (17), comprising a balloon catheter (10) having on a catheter tube (11) a balloon section (13) for receiving said vessel support (17) in the compressed state, and on each of both ends of the balloon section (13) a supporting member (15, 16) screening the compressed vessel support (17), each supporting member (15, 16) consisting of a ring in the shape of a truncated cone, the thinner end thereof being averted from the balloon section (13),
characterised in that
the balloon skin (12) surrounding the catheter tube (11) in the area of the balloon section (13) encloses the supporting members (15, 16).

2. Device according to claim 1, characterised in that at least one of the supporting members (15, 16) comprises a radiographic contrast medium.

3. Device according to claim 1 or 2, characterised in that the distance between the supporting members (15, 16) is from 20 to 30 mm in length.

4. Device according to any one of claims 1-3, characterised in that the diameter of the supporting members (15, 16) is by 0.12 to 0.6 mm larger than that of the balloon catheter (10) outside of the balloon section (13).

## Revendications

1. Dispositif pour l'application d'un support vasculaire (17) pouvant être élargi de façon permanente, comportant un cathéter à ballonnet (10) qui comporte, au niveau d'un tuyau (11) du cathéter, une section formant ballonnet (13) destinée à recevoir le support vasculaire (17) à l'état comprimé et, au niveau de chacune des deux extrémités de la section formant ballonnet (13), un élément d'appui respectif (15,16) qui protège le support vasculaire comprimé (17), et
dans lequel chaque élément d'appui (15,16) est constitué par un anneau de forme tronconique, dont l'extrémité la plus mince est tournée à l'opposé de la section formant ballonnet (13),
caractérisé en ce que la peau (12) du ballonnet, qui entoure le tuyau (11) du cathéter dans la zone de la section formant ballonnet (13), enveloppe les éléments d'appui (15,16).

2. Dispositif selon la revendication 1, caractérisé en ce qu'au moins l'un des éléments d'appui (15,16) fournit un contraste radiologique.

3. Dispositif selon la revendication 1 ou 2, caractérisé en ce que la distance entre les éléments d'appui (15,16) est de 20 à 30 mm.

4. Dispositif selon l'une des revendications 1 à 3, caractérisé en ce que le diamètre des éléments d'appui (15,16) est supérieur de 0,12 à 0,6 mm à celui du cathéter à ballonnet (10) à l'extérieur de la section formant ballonnet (13).
